# EUROPEAN PATENT SPECIFICATION

(11) **EP 3 167 043 B1**
(45) Date of publication and mention of the grant of the patent: **24.02.2021**
(21) Application number: 15818508.2
(22) Date of filing: 08.07.2015
(51) Int. Cl.: C12M 1/04, C12M 3/00, C12M 3/02, C12N 1/12, C12M 1/06

(54) **BIOREACTOR FOR PRODUCTION AND HARVESTING OF MICROALGAE**
BIOREAKTOR ZUR HERSTELLUNG UND GEWINNUNG VON MIKROALGEN
BIORÉACTEUR DE PRODUCTION ET DE RÉCOLTE DE MICRO-ALGUES

(30) Priority: 08.07.2014 NO 20140866; 08.08.2014 NO 20140975
(43) Date of publication of application: 17.05.2017
(73) Proprietor: Feeder International AS, 5160 LAKSEVÅG (NO)
(72) Inventor: KYRKJEBØ, Jan Erik, N-5238 Rådal (NO); GARVIK, Jan Fredrik, N-3830 Ulefoss (NO)
(74) Representative: Acapo AS
(86) International application number: PCT/NO2015/050125
(87) International publication number: WO 2016/007016

(56) References cited:
- WO-A1-2010/117726
- WO-A1-2014/006233
- WO-A2-2012/090203
- US-A1- 2012 288 917
- US-A1- 2012 329 147
- US-A1- 2014 273 171
- DATABASE WPI Week 201213 Thomson Scientific, London, GB; AN 2012-B82050 XP002777371, -& JP 2012 023979 A (HITACHI PLANT ENG&CONSTR CO LTD) 9 February 2012 (2012-02-09)
- DATABASE WPI Week 201177 Thomson Scientific, London, GB; AN 2011-P39218 XP002777372, & JP 2011 234677 A (NIKKEN SOHONSHA KK) 24 November 2011 (2011-11-24)
- DATABASE WPI Week 200901 Thomson Scientific, London, GB; AN 2009-A16796 XP002777373, & JP 2008 283937 A (DAIYA RIGHT JAPAN KK) 27 November 2008 (2008-11-27)
- CARVALHO AP . ET AL.: 'Microalgal reactors: A review of enclosed system designs and performances.' BIOTECNOLOGY PROG. vol. 22, 2006, pages 1490 - 1506, XP002640244

## Description

The present invention relates to a bioreactor for production and harvesting of microalgae, comprising a reactor basin in the form of a tank arranged to receive CO2 and water as well as algae, and which is equipped with at least one outlet for the harvested algal biomass.

From the patent literature, reference is made to WO 2010013998 A1 and WO 2010117726 A1. WO 2010013998 A1 relates to a bioreactor for cultivation and harvesting of algae, where the installation encompasses a rotating mixing mechanism that can also be used as a harvesting mechanism in that filter blades can push parts of the algal mass up into a collector. The whole of the mixing/harvesting mechanism can be moved along on the installation on a rail and thus cover the whole length of the installation. The reactor itself is inside a greenhouse and comprises several channels. Also described is a system for lighting and heating of the installation and the supply of CO2. WO 2010117726 A1 relates to a circular, floating bioreactor containing a mixing system with rotating rods.

Reference is also made to US 20110201102 A1, US 20120329147 A1 and US 8642326 B1. US 20110201102 A1 relates to a simple bioreactor for continuous cultivation of algae. It is comprised of a tank with associated pumps and pipelines for the supply of a nutrient solution, CO2 and a culture of algae. The tank is placed in a greenhouse-like structure and has lights mounted above the tank. In addition, the tank has a system for circulation of the algal solution in that a flow-director, comprising a flexible hose with holes for the bubbling in of CO2-rich water, is fastened in the bottom between two sidewalls. US 20120329147 A1 describes an installation for the cultivation of algae comprising several covered bioreactors in a floating structure. The installation has different pumps and pipes for supply and circulation of water, nutrients and algal culture. In addition, the bioreactors have a device for harvesting of the algal suspension that is dewatered and the harvested algae are led to a storage tank. US 8642326B1 relates to a system for the production and harvesting of algae in a reactor. The system comprises, in addition to a covered reactor tank, a turbulator for the mixing in of CO2 in the inlet water, guiding plates with integrated lights that ensure motion in the reactor, outlet channels for the harvesting, a float that measures the level in the reactor tank and also a dewatering installation for the harvested algae. The covered reactor also has systems for venting out the oxygen generated JP2012023979 discloses a photobioreactor (raceway pond) comprising a mixing means having a rotating beam and two different types of mixing units. The parallel direction stirring unit is a plate-shaped blade, the orthogonal direction stirring unit is a comb-shaped blade.

Several systems and devices for production of microalgae in reactors are known, such as raceway installations where the algae production itself takes place as well as a separate appliance for the harvesting of the algae.

These systems require particularly large areas and are often very costly. Furthermore, they will be particularly expensive to keep in operation because of cleaning and maintenance. They are also very complicated to construct and are very inefficient. In addition, they are subject to contaminations and will, as a rule, be built in places with a poor infrastructure and in connection with flue gas installations where one removes CO2 which, in addition to light, is a prerequisite to obtaining substantial growth of microalgae.

With the present invention one aims to solve the complicated and costly installation of today and at the same time have a closed system where one efficiently uses seawater as a CO2 source and where one, in addition, obtains a closed production and harvesting installation in one and the same unit.

One aims to combine the production and harvesting of algae in one and the same unit.

In addition, one aims to close the reactor into a closed room made from a glass material or other materials that let in light and at the same time avoid contaminations from the outside and which has a compact volume with low build measures.

This will make it possible to deliver algal oil and the product to an expanded market such as cosmetics, feed production for farming and to the pharmaceutical industry. In addition, one aims to utilise waste material from farming installations in a simple way and while, at the same time, compressed oxygen can be extracted from the production of the algae.

This can be solved by the reactor lid being airtight, but that one ensures for an utilisation of the oxygen that is generated from the production of the algae. This can take place in that the housing or the lid is connected to a fan with a closed pipe system instead of letting the oxygen out to the atmosphere.

Then, one can bring the oxygen to, for example, a generator and/or compressor that compresses the oxygen gas directly and which makes it possible to store this continuously in bottles and as a complete oxygen bottle battery and which in turn can be a further saleable product to the farming industry and otherwise to selective purposes related to flasks of oxygen.

This means the invention results in a very efficient production process and also one achieves an extra product in the form of oxygen ready for delivery from the production units themselves.

This will be a strength and will be a great resource which the fish farming industry uses in connection with delousing, invasion of jellyfish in the fish farming installations and as an additive and back up for closed and land-based farming installations.

Furthermore, according to the invention, a higher temperature can be realised in the algal reactor and in the production of algae, such as basic algal culture tanks that can also be installed on the reactor unit and for heating of the algal culture. This can be carried out, for example, in that the heat that is generated from the compression of the oxygen gas is heat exchanged with the seawater that is taken into the reactor and which then will serve to cool the oxygen production itself.

One aims at a technical solution that offers good control, low investment costs and an efficient, compact and maintenance friendly embodiment of the production of the algae, and also a method for production and harvesting of algae that is more continuous, hygienic and efficient compared to today's solutions.

Furthermore, the solution according to the invention will ensure that all fire regulations can be met very simply, at the same time as one tries to achieve an ergonomically functional solution for control of the production of the algae itself and the process around it.

Furthermore, it is, according to the invention, a considerable advantage that the harvesting duct is as large as possible at the outer edge where the algal biomass can peripherally glide across and out of the reactor in that the duct is slightly inclined and conical inwards towards a corner or an outlet in connection to dewatering units in the form of a decanter, centrifuge and possibly a press, to further be able to separate out pure algal oil as a saturated omega 3 acid which has a higher price than the algal biomass itself.

The algal biomass can be further utilised as jet fuel and animal feed.

The above mentioned objects are obtained with a bioreactor for production and harvesting of microalgae, comprising a reactor basin in the form of a tank arranged to receive CO2 and water as well as algae, and which is equipped with at least one outlet for the harvested algal biomass, characterised in that the reactor basin comprises a rotating beam equipped with one or more tiltable mixing grates and harvesting grates, as disclosed in claim 1.

Alternative embodiments are given in respective dependent claims.

Said mixing grates and harvesting grates can be tiltable and arranged to be parked in a position above the water in the reactor basin.

The rotating beam is preferably equipped with several arms, where said mixing grates and harvesting grates are mounted on respective arms. Alternatively said mixing grates and harvesting grates can be mounted on the same arm.

A bearing assembly can be mounted centrally in a gangway that extends across the reactor basin, and where the rotating beam is suspended over the reactor basin in the bearing assembly.

Furthermore, the ends of the rotating beam can be equipped with motorised drive wheels that roll on an upper, horizontal surface section adjoining the reactor basin.

The harvesting grates are preferably formed as a fine grate or grid to let through the water but not the biomass, and can comprise a lower section that scrapes against the bottom of the reactor basin to bring along the algal mass.

The mixing grates are preferably formed as a rough grid to let through water and algal mass and are arranged to mix or stir the mass of algae in the reactor basin.

The mixing grates can be equipped with a brush or the like on a lower section and possibly on a side edge for the cleaning of the sidewall and bottom of the reactor basin.

Furthermore, the mixing grates can be equipped with a number of diffusors for the injection of inert air in the reactor basin water, where the inert air promotes growth of the algal biomass.

The reactor basin can comprise one or more inclined harvesting ducts that extend from an area adjoining the centre of the reactor basin and out past the outer circumference of the reactor basin, where the harvested biomass of algae is arranged to flow out via the harvesting duct and to a collecting tank.

The harvesting duct can be comprised of an elevated section in an area adjoining the surface of the water in the reactor basin where, in the front of the harvesting duct, a first section of the bottom of the reactor basin runs at an angle or in an arch up towards the harvesting duct and, after the harvesting duct, a second section runs in an arch down to the bottom again.

A frame for lights can be mounted to the beam and can be driven around together with this, where a number of lights in the form of light rods that hangs down into the reactor basin to stimulate production of algae are mounted to the light frame.

Furthermore, the reactor basin can be covered by a roof or lid so that a closed room over the reactor basin is set up, where the roof is transparent and made from a plastic or glass to let through light that stimulates the production of algae.

The bioreactor can be mounted in a floating construction that is equipped with a float collar so that the construction is arranged to float in or on a water surface, and also that the floating construction contains pumps and other equipment for the operation of the bioreactor.

The invention shall now be described in more detail with the help of the enclosed figures, in which:
Figure 1 shows a floating construction with a bioreactor according to the invention.
Figure 2 shows a partial section through the construction shown in figure 1.
Figure 3 shows in more detail the reactor shown in figure 2.
Figure 4 shows an outline of a closed bioreactor according to the invention, seen from above.
Figures 5 - 7 show more details of the bioreactor according to the invention, seen from above.
Figures 8 - 9 show mixing grates that are part of the bioreactor according to the invention.
Figures 10 - 11 show harvesting grates that are part of the bioreactor according to the invention.
Figure 12 shows in more detail a section of a mixing grate mounted in the reactor basin.

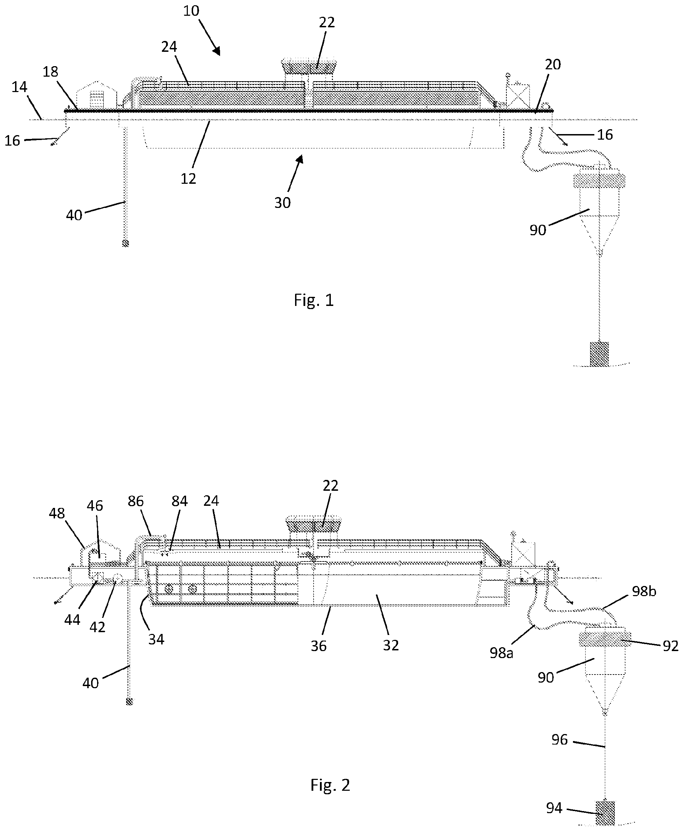

As shown in figure 1, an embodiment of the invention comprises a floating construction 10 that can be equipped with a float collar 12 so that the construction is arranged to float in or on a water surface 14. For the anchoring, the float collar 12 can be equipped with anchoring lines 16 that run down into the water to an anchoring point (not shown). Thus, the floating construction 10 can be anchored in the same way as an ordinary floating fish farming installation. Furthermore, the floating construction 10 can comprise one or more quays 18,20 where a vessel can be moored and which can contain pumps and other equipment for the operation of a bioreactor 30 mounted in the floating construction.

The bioreactor 30 is shown in more detail in figure 2 and comprises a reactor basin 32 with a sidewall 34 and a bottom 36 so that a water-filled tank is formed. Meant by the expression "bioreactor" is a reactor basin and associated pumps and equipment for the operation of the production of algae, and also other equipment. The reactor basin 32 is filled with water to a given level, be it higher, lower or corresponding to the level of the surface of the surrounding water 14 and is connected to an inlet pipe 40 for the supply of water. The water can be pumped up with the help of a pump 42 and is led on to a filter system 44. Thereafter, the water is led to a production installation 46 for the supply of the basic culture of algae, i.e. the starting culture which is led further to the reactor basin 32. The pump 42, or the pumps, and the filter system 44 can be placed in a lower part of the quay 18 while the production installation 46 can be placed in a housing 48 on the same quay 18.

It is preferred that a number of gangways 24 run across the reactor basin 32 that meet at a central point above the reactor basin. A central control room 22 can be placed at the central point. The gangways and the control room ensure a clear and ergonometric operation of the bioreactor. The water and algae that are supplied to the reactor basin 32 are led to the production installation 46 via pipelines 50 or tubes mounted on the gangway 24, or water is pumped directly from the pump 42 and the filter system 44 via the pipe(s) 50 to the reactor basin 32.

The gangway 24, or more specifically, the central point where the gangways meet, will also function as a bearing for a rotating beam 54, where the beam is mounted in a bearing assembly 52. For the operation of the beam 54, the bearing assembly 52 can be motorised, but it is more preferred that the ends 56 of the beam are equipped with motorised drive wheels 58 that roll on an upper, horizontal surface section 38 adjoining the reactor basin 32. This means that the construction is very simple to build in that the bearing assembly 52 can have a very small diameter and the beam 54 is supported and is driven round at a controlled speed. Water and any algae that are supplied through the pipeline 50 can be distributed in the reactor basin 32 via the bearing assembly 52. Correspondingly, fertiliser or nutrient salts can also be supplied via the bearing assembly 52. Furthermore, the bearing assembly 52 can comprise an electric slip ring for the transfer of electrical current to a number of mixing grates 60 and harvesting grates 62 mounted on the rotating beam 54. The mixing grates 60 and the harvesting grates 62 will be explained in more detail later.

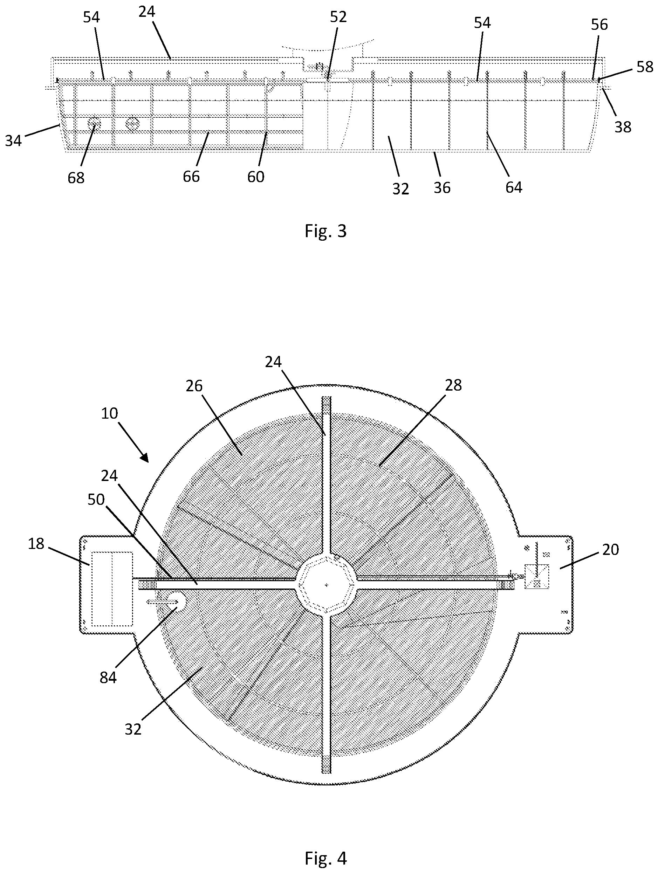

Figure 4 shows the floating construction 10 with the bioreactor 30 seen from above, and, as can be seen, has a circular shape in the embodiment shown. With a rotating beam 54 it is preferred that the reactor basin 32 is circular, but the reactor basin can naturally have other shapes also. Thereby, the gangways 22 meet as shown in the centre of the circular shape, and where, as mentioned, the control room 22 can be placed. The reactor basin 32 is preferably covered by a roof 26 or a lid so that a closed room is provided over the reactor basin, which means that the bottom 36, wall 34 and roof 26 of the reactor basin provide a closed space. The roof 26 is preferably transparent and made from plastic or glass to let though light which stimulates the production of the algae. In that the transparent roof 26 is mounted as low as possible, and preferentially at a level under the gangway 24 and the control room 22, this results in the solution according to the invention floating very low in the water 14, which again is an advantage aesthetically and also a considerably cheaper solution in addition to that the production occurs in an environmentally friendly and clean way. Furthermore, less maintenance is required with this embodiment example. Additionally, this embodiment example is subjected to smaller wind forces which can simplify its anchoring and expand the area where the production can take place.

The roof 26 can be equipped with venting hatches in cases where suction fans 84 stop or a too high oxygen level with respect to the requirements by the authorities or other bodies should be reached. Then, these hatches can be hinged and be able to be opened automatically by a twist motor that opens the venting hatches at too high oxygen levels. This ensures an explosion free bioreactor which can thereby be given general approval with respect to explosion safety. The fan 84 can be driven by an electric motor.

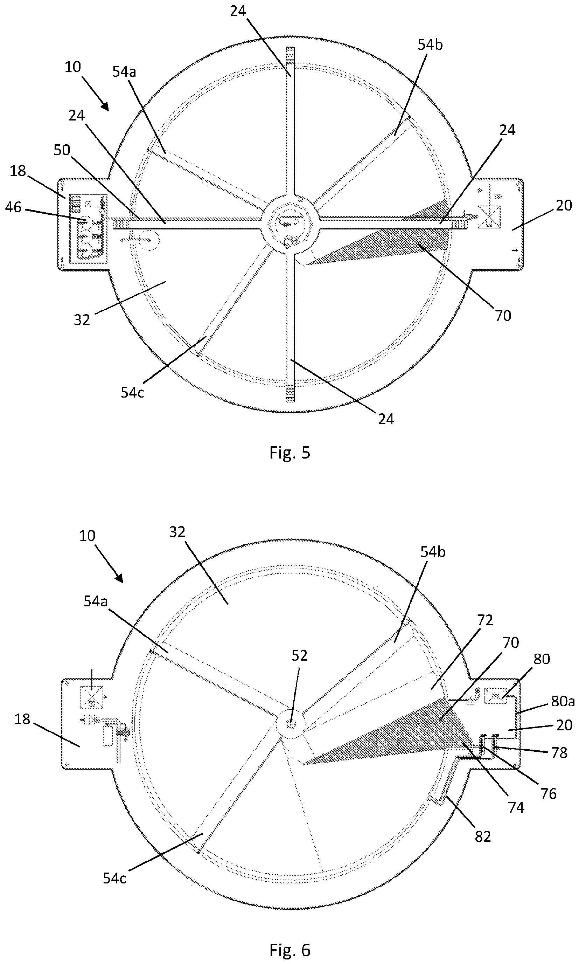

The rotating beam 54 can (as shown in figure 3) have two arms. In a more preferred embodiment, such as, for example, shown in figures 5 and 6, the beam can be comprised of several arms, in this case three arms 54a,54b,54c, where all the arms are mounted as explained in the bearing assembly 52 and are driven round by the drive wheels 58. With the use of several arms, a smoother and better mixing of the algal mass in the reactor basin 32 is ensured, and also a faster and better harvesting of the algal mass.

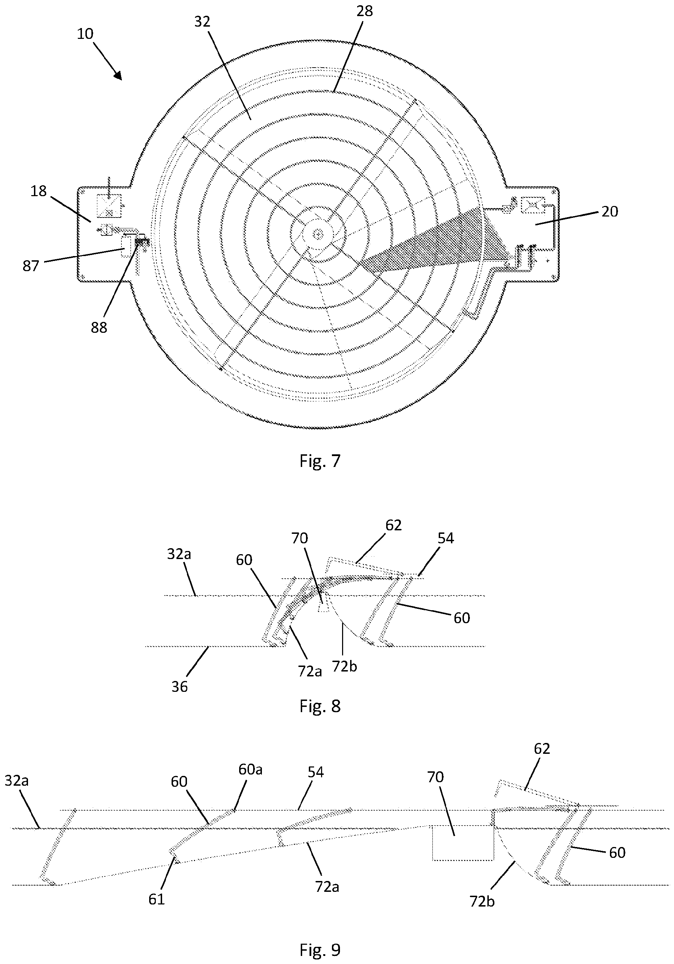

As shown in figures 4 and 7, and for that matter figure 3, a frame 28 for lights can be mounted to the beam 54 (or the arms 54a,54b,54c of the beam) and be driven round with this. The frame 28 is preferably mounted above the beam 54, but below the gangway(s) 24. The frame can also be mounted below the beam 54. The frame 28 can comprise a number of circular rings, where the rings are mounted radially outside each other with a steadily increasing diameter. However, the frame 28 can, if required, be independently mounted in the bearing assembly 52 and be driven round on its own independently of the rotation of the beam 54.

A number of lights are mounted on the light frame 28 to stimulate the production of algae in the reactor basin 32. The lights can be in the form of light rods 64 that hangs down into the reactor basin 32. The lights can also be mounted on the mixing grates 60.

For harvesting of the mass of algae, the reactor basin 32 preferably comprises one or more harvesting ducts 70, in the embodiment shown one duct. The harvesting duct 70 preferably extends from an area adjoining the centre of the reactor basin 32 and radially out towards and past the outer circumference of the reactor basin 32, and possibly with a steadily increasing cross section. Furthermore, the harvesting duct 70 is, for example, placed on an elevated section 72 of the bottom of the reactor basin 32, and so that an upper part of the harvesting duct 70 lies in a surface zone 32a in the basin 32. The harvesting duct 70, or the bottom of the harvesting duct, leans or inclines radially upwards, out and past the outer circumference of the reactor basin 32 and ends up in a collecting tank 74. In this way a very flexible and efficient production and harvesting of the algal biomass can be obtained in one and the same unit. The harvesting becomes even more efficient in that the duct 70 is gently inclined outwards and down from the centre and that the duct thereafter is angled inwards, as shown in figure 6. In this way the algal biomass can run freely outwards and down in the harvesting duct 70 and down into the collecting tank 74, which in turn can be connected to a pump and/or a centrifuge and or a decanter 76. A press 78 can be used to dewater the algal biomass and at the same time separate out pure algal oil that can be stored in a submersible tank 90. At the same time, a residual algal biomass can be produced that can be transported to and stored in a tank 80 via a pipeline 80a. This can in a way be a pump pipeline for the flow or be a transporter screw. Additionally, the excess biomass can be further pumped in return to the reactor basin 32 via the lines 82 to be used again in the algae production. In this way an attractive product in the form of algal oil that is highly priced can be produced and where the production becomes particularly efficient where no culture of algae is wasted.

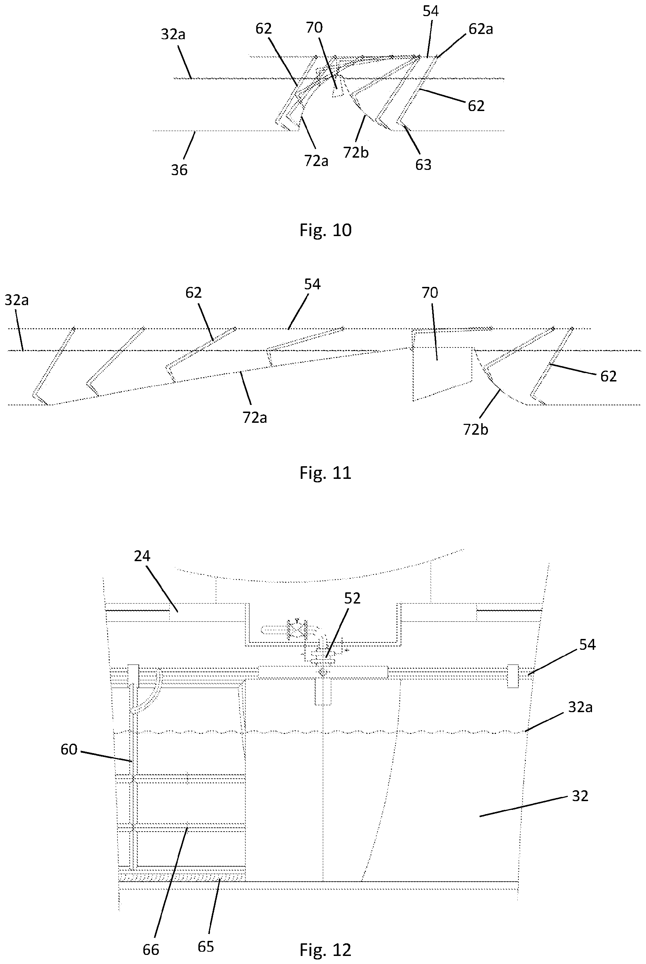

As mentioned, the present invention comprises a number of mixing grates 60 and a number of harvesting grates 62 mounted to the rotating beam 54, or more specifically to the arms 54a,54b,54c. In the figures 10 and 11, the harvesting grates 62 are shown illustratively fastened to the beam 54, and comprise a grate or grid or the like that is tiltably fastened to the beam 54 in a mounting point 62a. The harvesting grates 62 can be tiltable and be parked in an upper position above the water in the reactor basin with the help of a motor, cylinder or cogwheel/cogged rod system that can be controlled from, for example, the control room 22. The harvesting grates 62 can, as illustrated, be formed in an L-shape or the like with a lower part 63 that scrapes against the bottom of the reactor basin 32 to bring along the mass of algae. The remaining part of the harvesting grate is further arranged to catch and bring along the algal mass, but is preferably formed so that it lets through water only but not the mass of algae and can, for that reason, be equipped with, for example, a fine mesh net or grid. The harvesting grate 62 can be comprised of one or more grates and can be arranged along the whole, or part, of the length of the beam 54.

Consequently, the harvesting grates 62 are used to lead the algal mass to the harvesting duct 70. To simplify this work, an area on each side of the harvesting duct 70 can comprise the elevated section 72 such as the sections 72a,72b shown in the figures 10 and 11. At the front of the harvesting duct 70, the first section 72a of the bottom 36 of the reactor basin 32 runs at an angle or arched upwards to the harvesting duct 70. The line 32a illustrates the water level in the reactor basin. After the harvesting duct 70, the second section 72b runs in an arch down to the bottom 36 again. Figure 10 illustrates the elevated sections 72a,72b near the centre of the reactor basin 32, while figure 11 illustrates the elevated sections 72a,72b peripherally from the centre of the reactor basin 32. So that the harvesting grates 62 shall not fall down in, or get stuck in the harvesting duct 70, the harvesting duct is preferably equipped on the top side with a course grid, leading rod, or the like which the lower part 63 of the harvesting grates 62 can glide across.

The harvesting grates 62 can, when they are not in use, be parked in an upper position, as shown in figure 9.

In the figures 8 and 9 the mixing grates 60 are shown illustratively fastened to the beam 54 and comprise a grate, grid or the like that is tiltably fastened to the beam 54 via a mounting point 60a. The mixing grates 60 can be tiltable and be parked in an upper position above the water in the reactor basin with the help of a motor, a cylinder or a cogwheel/ cogged rod system that can be controlled from, for example, the control room 22, corresponding to the harvesting grates 62 and can, as illustrated, be formed in an L-shape or the like with a bent lower part 61 against the bottom of the reactor basin 32 to mix or stir the algal mass in the reactor basin 32. The remaining part of the mixing grate is further arranged to mix or stir the algal mass and is formed so that it lets through water and algal mass. For that reason, the mixing grates 60 can be equipped with, or formed as, a coarse grid, such as shown in the figures 3 or 12 and also have a straight shape.

The mixing grates 60 and the harvesting grates 62 can be tiltably mounted to respective parts of the beam 54 or the arms 54a,54b,54c so that a number of mixing grates 60 are mounted on one arm, while a number of harvesting grates 62 are mounted on another arm. The grates 60,62 that are not in use are tilted up. Alternatively, a number of mixing grates 60 and a number of harvesting grates 62 can be mounted on the same arm or beam. In the latter case, the harvesting grates 62 can be tilted up, as shown in the figures 8 and 9, while the mixing grates 60 are in use. Inversely, the mixing grates 60 can be tilted up, readily in the opposite way of the harvesting grates, and the harvesting grates 62 are dropped down in the reactor basin 32 for the harvesting of the algal mass as explained earlier, while the mixing grates 60 are tilted up.

The mixing grates 60 can also be equipped with a brush 65 on a lower part and possibly on its side edge so that the bottom 32 and the side wall 34 of the reactor basin can be cleaned continuously and thereby constitute a more or less maintenance free solution. In this way the production can take place both day and night in a more or less automatic process and in that the reactor basin itself does not need to be emptied or cleaned manually.

Furthermore, the mixing grates 60 can be equipped with nozzles/diffusors 66 for injection of air into the reactor basin 32. The air that is injected in the reactor basin 32 can be inert air, i.e. air with a reduced oxygen content, that is pumped in with the help of an air pump (not shown). The mixing grates 60 can also be equipped with stirring means in the form of, for example, propeller-like appliances 68, to increase the production of algae. This ensures stirring the reactor content continuously for the whole 24 hour period and prevents sedimentation of the algae biomass in the reactor.

There are, in the main, three different technologies that are used for the production of inert air, membrane technology, PSA technology (Pressure Swing Absorption) and VSA technology (Vacuum Swing Adsorption). The inert air generator, whatever the technology, separates out oxygen from standard air. The remaining oxygen depleted inert air is supplied/dosed into the bioreactor 30. The bioreactor 30 can provide stirring via its air pump and aeration of the culture of microalgae by supplying inert air (ultrapure air) continuously according to need via diffusors 66 mounted on the mixing grates 60 or in the bottom of the reactor basin 32 so that the oxygen level is held within given minimum/maximum limit values for inert air. The oxygen that is generated during the photosynthesis in the reactor basin 32 is initially a by-product. Inert air used in a bioreactor 30 to increase the growth of the algal biomass is appropriate if the loss of oxygen is growth promoting or growth preventing. The Enzyme Rubisco regulates the uptake of CO2 and works best at low temperatures. This indicates that Nordic/arctic diatoms are very well suited to "carbon capture". Briefly, it is the enzyme rubisco that takes up the CO2 and O2. To obtain a rapid and substantial CO2 uptake, it is therefore important that the microalgae take up relatively little O2 in relation to carbon dioxide CO2. This means that northerly algal species that are adapted to low temperatures are more efficient in absorbing CO2 than (southerly) species that grow at high temperatures. This is, in turn, reinforced by the algal Rubisco efficiency which binds carbon dioxide. Thereby, the use of inert air can lead to increased growth of algae.

In this way one obtains a continuous and flexible production and harvesting that can be automated and controlled continuously. By harvesting a part of the culture and refilling with a new growth medium, one can keep the culture in the growth phase that gives the maximum yield. The process can also be made continuous by pumping in growth medium and harvesting the algae in the outlet from the culture in the bioreactor 30. In this way one can, in principle, obtain a constant production for an unlimited time. In that the algae are kept in the same growth phase the whole time, the composition and quality of the algal biomass is relatively constant.

The production installation 46 can also use inert air in a separate cultivation station/marine biobank for refilling of marine microorganisms in the reactor basin 32. Cultivation chambers that are continuously supplied with ultrapure air to promote the growth rate can be used in the cultivation station. When full saturation is achieved in the chamber the algal culture is pumped out into the reactor. The production in the cultivation station takes place under optimal nutrient feeding and can be driven as a continuous operation that has a dilution speed that balances the outtake of algal biomass.

As mentioned, the bioreactor 30 can be equipped with suction fans 84. These can also be used to suck out separated oxygen from the bioreactor and can be led via a pipeline 86 to a compressor 88 and possibly via an oxygen generator that compresses the free oxygen to a higher pressure that can be stored in a pressure tank 87 (figure 7) for later filling of gas bottles which in turn can be used as oxygen for, for example, fish farming installations along the coast. The compressor 88 can be surrounded by a cooler, the cooling water of which is connected to the seawater inlet pipe 40 which thereby ensures that the seawater which is pumped into the reactor 30 is not undercooled which in turn can damage the value of the algae production. In this way both production and storage of compressed oxygen and heat taken in with the seawater, can be achieved.

The submersible tank 90 for the storage of produced algal biomass can, as shown, comprise a float collar 92 that provides buoyancy and can be anchored to the seabed via a cable 96 and an anchoring element 94. Furthermore, the tank 90 can be connected to the algae production installation in the quay 20 via pipeline 98a,98b to receive and export algal biomass.

## Claims

1. Bioreactor (30) for production and harvesting of microalgae, comprising a reactor basin (32) in the form of a tank arranged to receive CO₂ and water, as well as algae, and which is equipped with at least one outlet for harvested algal biomass,
**characterised in that** the reactor basin (32) comprises a horizontally rotating beam (54) equipped with one or more tiltable mixing grates (60) and harvesting grates (62), wherein the harvesting grates (62) are formed as a fine grate or grid to let through water but not the algal mass, and comprise a lower part (63) that scrapes against the bottom of the reactor basin (32) to bring along the mass of algae, and the mixing grates (60) are formed as a coarse grid to let through water and the algal mass and are arranged to mix or stir the mass of algae in the reactor basin (32).

2. Bioreactor according to claim 1, **characterised in that** said mixing grates (60) and harvesting grates (62) are tiltable and arranged to be parked in a position above the water (32a) in the reactor basin (32).

3. Bioreactor according to claim 1, **characterised in that** the rotating beam (54) is equipped with several arms (54a,54b,54c), where said mixing grates (60) and harvesting grates (62) are mounted together on respective arms.

4. Bioreactor according to claim 1, **characterised in that** the rotating beam (54) is equipped with several arms (54a,54b,54c), where said mixing grates (60) and harvesting grates (62) are mounted separately on respective arms.

5. Bioreactor according to claim 1, **characterised in that** a bearing assembly (52) is mounted centrally in a walkway (24) that extends across the reactor basin (32) and that the rotating beam (54) is suspended above the reactor basin (32) in the bearing assembly (52).

6. Bioreactor according to claim 1, **characterised in that** the ends (56) of the rotating beam (54) are equipped with motorised drive wheels (58) that roll on an upper horizontal surface section (38) adjoining the reactor basin (32).

7. Bioreactor according to claim 1, **characterised in that** the mixing grates (60) are equipped with a brush (65) or the like on a lower part, and possibly on a side edge, for cleaning of the bottom and side wall of the reactor basin (32).

8. Bioreactor according to claim 1, **characterised in that** the mixing grates (60) are equipped with a number of diffusors (66) for injection of inert air into the water in the reactor basin (32), where the inert air is growth promoting for the algal biomass.

9. Bioreactor according to claim 1, **characterised in that** the reactor basin (32) is comprised of one or more inclined harvesting ducts (70) running in the radial direction, and which extend from an area adjoining the centre of the reactor basin (32) and out past the outer circumference of the reactor basin (32), where the harvested mass of algae is arranged to flow out through the harvesting duct (70) and to a collecting tank (74).

10. Bioreactor according to claim 9, **characterised in that** the harvesting duct (70) comprises an elevated section (72) in an area adjoining the water surface of the reactor basin (32), where, in the front of the harvesting duct (70), a first section (72a) of the bottom (36) of the reactor basin (32) runs at an angle or in an arch upwards to the harvesting duct (70), and after the harvesting duct (70), a second section (72b) runs in an arch downwards to the bottom (36) again.

11. Bioreactor according to claim 1, **characterised in that** a frame (28) for lights is mounted to the beam (54) and is driven round with this, where a number of lights in the form of light rods (64) that hang down into the reactor basin (32) to stimulate the production of algae is mounted to the light frame (28).

12. Bioreactor according to claim 1, **characterised in that** the reactor basin (32) is covered with a roof (26) or lid so that a closed room is created above the reactor basin, where the roof (26) is transparent and made from plastic or glass to let through light that stimulates the production of algae.

13. Bioreactor according to claim 1, **characterised in that** the bioreactor (30) is mounted in a floating construction (10), and which is equipped with a float collar (12) so that the construction is arranged to float in or on a water surface (14), and also that the floating construction (10) contains pumps and other equipment for the operation of the bioreactor (30).

## Patentansprüche

1. Bioreaktor (30) zur Produktion und Ernte von Mikroalgen, umfassend ein Reaktorbecken (32) in Form eines Behälters, das zur Aufnahme von CO₂ und Wasser sowie Algen eingerichtet ist, und das mit mindestens einem Auslass für geerntete Algenbiomasse ausgestattet ist,
**dadurch gekennzeichnet, dass** das Reaktorbecken (32) einen horizontal rotierenden Balken (54) umfasst, der mit einem oder mehreren kippbaren Mischgittern (60) und Erntegittern (62) ausgestattet ist, wobei die Erntegitter (62) als ein feines Gitter oder Raster ausgebildet sind, um Wasser, jedoch nicht die Algenmasse durchzulassen, und einen unteren Abschnitt (63) umfasst, der gegen den Boden des Reaktorbeckens (32) schabt, um die Algenmasse mitzunehmen, und die Mischgitter (60) als ein grobes Raster ausgebildet sind, um Wasser und die Algenmasse durchzulassen, und dazu eingerichtet sind, die Algenmasse in dem Reaktorbecken (32) zu mischen oder zu rühren.

2. Bioreaktor gemäß Anspruch 1, **dadurch gekennzeichnet, dass** die Mischgitter (60) und Erntegitter (62) kippbar und dazu eingerichtet sind, in einer Position oberhalb des Wassers (32a) im Reaktorbecken (32) abgestellt zu werden.

3. Bioreaktor gemäß Anspruch 1, **dadurch gekennzeichnet, dass** der rotierende Balken (54) mit mehreren Armen (54a, 54b, 54c) ausgestattet ist, wobei die Mischgitter (60) und Erntegitter (62) gemeinsam an jeweiligen Armen angebracht sind.

4. Bioreaktor gemäß Anspruch 1, **dadurch gekennzeichnet, dass** der rotierende Balken (54) mit mehreren Armen (54a, 54b, 54c) ausgestattet ist, wobei die Mischgitter (60) und Erntegitter (62) separat an jeweiligen Armen angebracht sind.

5. Bioreaktor gemäß Anspruch 1, **dadurch gekennzeichnet, dass** eine Lageranordnung (52) mittig in einem sich über das Reaktorbecken (32) erstreckenden Steg (24) angebracht ist und dass der rotierende Balken (54) oberhalb des Reaktorbeckens (32) in der Lageranordnung (52) aufgehängt ist.

6. Bioreaktor gemäß Anspruch 1, **dadurch gekennzeichnet, dass** die Enden (56) des rotierenden Balkens (54) mit motorisierten Antriebsrädern (58) ausgestattet sind, die auf einem oberen horizontalen an das Reaktorbecken (32) angrenzenden Flächenabschnitt (38) rollen.

7. Bioreaktor gemäß Anspruch 1, **dadurch gekennzeichnet, dass** die Mischgitter (60) an einem unteren Abschnitt und gegebenenfalls an einer seitlichen Kante mit einer Bürste (65) oder dergleichen zur Reinigung des Bodens und der Seitenwand des Reaktorbeckens (32) ausgestattet sind.

8. Bioreaktor gemäß Anspruch 1, **dadurch gekennzeichnet, dass** die Mischgitter (60) mit einer Anzahl von Diffusoren (66) zum Einblasen von Inertluft in das Wasser im Reaktorbecken (32) ausgestattet sind, wobei die Inertluft wachstumsfördernd für die Algenbiomasse ist.

9. Bioreaktor gemäß Anspruch 1, **dadurch gekennzeichnet, dass** das Reaktorbecken (32) einen oder mehrere schräg in radialer Richtung verlaufende Erntekanäle (70) umfasst, und die sich von einem an das Zentrum des Reaktorbeckens (32) angrenzenden Bereich und über den Außenumfang des Reaktorbeckens (32) hinaus erstrecken, wobei die geerntete Algenmasse durch den Erntekanal (70) und zu einem Sammelbehälter (74) abfließen kann.

10. Bioreaktor gemäß Anspruch 9, **dadurch gekennzeichnet, dass** der Erntekanal (70) in einem an die Wasseroberfläche des Reaktorbeckens (32) angrenzenden Bereich einen erhöhten Abschnitt (72) umfasst, wobei vor dem Erntekanal (70) ein erster Abschnitt (72a) des Bodens (36) des Reaktorbeckens (32) in einem Winkel oder bogenförmig nach oben zum Erntekanal (70) verläuft, und nach dem Erntekanal (70) ein zweiter Abschnitt (72b) bogenförmig wieder nach unten zum Boden (36) verläuft.

11. Bioreaktor gemäß Anspruch 1, **dadurch gekennzeichnet, dass** an dem Balken (54) ein Gestell (28) für Leuchten angebracht ist und mit diesem umherbewegt wird, wobei an dem Gestell (28) für Leuchten eine Anzahl von Leuchten in Form von Lichtstäben (64) angebracht ist, die in das Reaktorbecken (32) herabhängen, um die Algenproduktion zu stimulieren.

12. Bioreaktor gemäß Anspruch 1, **dadurch gekennzeichnet, dass** das Reaktorbecken (32) mit einem Dach (26) oder Deckel bedeckt ist, so dass ein geschlossener Raum über dem Reaktorbecken erzeugt wird, wobei das Dach (26) transparent ist und aus Kunststoff oder Glas besteht, um Licht durchzulassen, das die Produktion von Algen stimuliert.

13. Bioreaktor gemäß Anspruch 1, **dadurch gekennzeichnet, dass** der Bioreaktor (30) in einer schwimmenden Konstruktion (10) angebracht ist, und die mit einem Schwimmkragen (12) ausgestattet ist, so dass die Konstruktion dazu eingerichtet ist, in oder auf einer Wasseroberfläche (14) zu schwimmen, und wobei die schwimmende Konstruktion (10) Pumpen und andere Ausrüstung für den Betrieb des Bioreaktors (30) enthält.

## Revendications

1. Bioréacteur (30) pour la production et la récolte de microalgues, comprenant un bassin de réacteur (32) sous la forme d'une cuve agencée pour recevoir du CO₂ et de l'eau, ainsi que des algues, et qui est équipée d'au moins une sortie pour de la biomasse algale récoltée,
**caractérisé en ce que** le bassin de réacteur (32) comprend une poutre tournant horizontalement (54) équipée d'une ou plusieurs grilles de mélange (60) et de grilles de récolte (62) inclinables, dans lequel les grilles de récolte (62) sont constituées sous forme d'un grillage ou d'une grille fine laissant passer l'eau mais pas la masse algale, et comprennent une partie inférieure (63) qui frotte contre le fond du bassin de réacteur (32) pour ramener la masse d'algues, et les grilles de mélange (60) sont constituées sous forme de grilles grossières pour laisser passer l'eau et la masse algale et sont agencées pour mélanger ou agiter la masse des algues dans le bassin de réacteur (32).

2. Bioréacteur selon la revendication 1, **caractérisé en ce que** lesdites grilles de mélange (60) et lesdites grilles de récolte (62) sont inclinables et sont conçues pour être parquées dans une position au-dessus de l'eau (32a) dans le bassin de réacteur (32).

3. Bioréacteur selon la revendication 1, **caractérisé en ce que** la poutre rotative (54) est équipée de plusieurs bras (54a, 54b, 54c), dans lequel lesdites grilles de mélange (60) et lesdites grilles de récolte (62) sont montées ensemble sur des bras respectifs.

4. Bioréacteur selon la revendication 1, **caractérisé en ce que** la poutre rotative (54) est équipée de plusieurs bras (54a, 54b, 54c), dans lequel lesdites grilles de mélange (60) et lesdites grilles de récolte (62) sont montées séparément sur des bras respectifs.

5. Bioréacteur selon la revendication 1, **caractérisé en ce qu'**un ensemble de palier (52) est monté centralement dans une passerelle (24) qui s'étend à travers le bassin de réacteur (32) et **en ce que** la poutre rotative (54) est suspendue au-dessus du bassin de réacteur (32) dans l'ensemble de palier (52).

6. Bioréacteur selon la revendication 1, **caractérisé en ce que** les extrémités (56) de la poutre rotative (54) sont équipées de roues d'entraînement motorisées (58) qui roulent sur une section de surface horizontale supérieure (38) jouxtant le bassin de réacteur (32).

7. Bioréacteur selon la revendication 1, **caractérisé en ce que** les grilles de mélange (60) sont équipées d'une brosse (65) ou similaire sur une partie inférieure, et éventuellement sur un bord latéral, pour nettoyer le fond et la paroi latérale du bassin de réacteur (32).

8. Bioréacteur selon la revendication 1, **caractérisé en ce que** les grilles de mélange (60) sont équipées d'un certain nombre de diffuseurs (66) pour injecter de l'air inerte dans l'eau dans le bassin de réacteur (32), dans lequel l'air inerte favorise la croissance de la biomasse algale.

9. Bioréacteur selon la revendication 1, **caractérisé en ce que** le bassin de réacteur (32) est constitué d'un ou plusieurs conduits de récolte inclinés (70) s'étendant dans la direction radiale, et qui s'étendent à partir d'une région attenante au centre du bassin de réacteur (32), hors et au-delà de la circonférence extérieure du bassin de réacteur (32), dans lequel la masse récoltée d'algues est agencée pour s'écouler à l'extérieur via le conduit de récolte (70) et jusqu'à une cuve de collecte (74).

10. Bioréacteur selon la revendication 9, **caractérisé en ce que** le conduit de récolte (70) comprend une section haute (72) dans une région attenante à la surface de l'eau du bassin de réacteur (32), dans lequel, à l'avant du conduit de récolte (70), une première section (72a) du fond (36) du bassin de réacteur (32) s'étend selon un angle ou selon une voûte vers le haut vers le conduit de récolte (70), et après le conduit de récolte (70), une seconde section (72b) s'étend selon une voûte vers le bas à nouveau jusqu'au fond (36).

11. Bioréacteur selon la revendication 1, **caractérisé en ce qu'**un cadre (28) pour des lampes est monté sur la poutre (54) et est entraîné pour tourner avec celle-ci, dans lequel un certain nombre de lampes en forme de barres lumineuses (64) qui sont suspendues dans le bassin de réacteur (32) pour stimuler la production d'algues sont montées sur le cadre lumineux (28).

12. Bioréacteur selon la revendication 1, **caractérisé en ce que** le bassin de réacteur (32) est recouvert d'un toit (26) ou d'un couvercle de façon à créer un espace fermé au-dessus du bassin de réacteur, dans lequel le toit (26) est transparent et constitué de plastique ou de verre pour laisser passer la lumière qui stimule la production d'algues.

13. Bioréacteur selon la revendication 1, **caractérisé en ce que** le bioréacteur (30) est monté sur une construction flottante (10), et qui est équipé d'un collier flottant (12), de telle manière que la construction est adaptée pour flotter dans ou sur une surface d'eau (14), et aussi que la construction flottante (10) contient des pompes ou d'autres équipements pour le fonctionnement du bioréacteur (30).
